# EUROPEAN PATENT SPECIFICATION

(11) **EP 0 935 752 B1**
(45) Date of publication and mention of the grant of the patent: **21.11.2001**
(21) Application number: 96933065.3
(22) Date of filing: 20.09.1996
(51) Int. Cl.: G01N 33/50, G01N 33/74

(54) **METHODS FOR IN VITRO DETERMINATION OF ERYTHROPOIETIN BIOACTIVITY**
METHODE ZUR IN VITRO-BESTIMMUNG DER BIOAKTIVITÄT VON ERYTHROPOIETIN
PROCEDES DE DETERMINATION IN VITRO DE LA BIOACTIVITE DE L'ERYTHROPOIETINE

(43) Date of publication of application: 18.08.1999
(73) Proprietor: Ortho-McNeil Pharmaceutical, Inc., Raritan, NJ 08869-0606 (US)
(72) Inventor: LISI, Peter, J., Flemington, NJ 08822 (US); GLENN, Jeffrey, K., Middlesex, NJ 08846 (US); SO, Chi-Kwong, Somerville, NJ 08876 (US)
(74) Representative: Mercer, Christopher Paul
(86) International application number: US9615049
(87) International publication number: WO9812558

(56) References cited:
- EP-A- 0 183 404
- EP-A- 0 358 463
- WO-A-92/06116
- PATENT ABSTRACTS OF JAPAN vol. 014, no. 522 (C-0778), 15 November 1990 & JP 02 215398 A (SNOW BRAND MILK PROD CO LTD), 28 August 1990,
- THE JOURNAL OF BIOLOGICAL CHEMISTRY, vol. 265, no. 21, 1990, pages 12127-12130, XP002031640 M. TAKEUCHI ET AL.: "Role of sugar chains in the in vitro biological activity of human erythropoietin produced in recombinant chinese Hamster ovary cells."

## Description

### FIELD OF THE INVENTION

Erythropoietin (EPO) is a glycoprotein hormone which stimulates the maturation of erythroid precursor cells and thereby regulates the production of erythrocytes in mammals. The purification of human EPO (hEPO) and the cloning of the EPO gene have led to the commercial production of recombinant hEPO, which has been successfully used in the treatment of patients with anemia due to renal failure, and is clinically useful in the treatment of other anemias. Present methods of quantitating the biologically active EPO in a sample, for example a bulk drug formulation, are cumbersome, expensive and time-consuming. The present invention provides a safe, rapid and relatively inexpensive method of measuring the in vivo bioactivity of EPO, and kits for measuring EPO bioactivity.

### BACKGROUND OF THE INVENTION

EPO is a glycoprotein hormone which stimulates the proliferation, differentiation and maturation of erythroid precursor cells to mature red blood cells. EPO has been purified (Miyake et al., (1977) J. Biol. Chem. 252: 5558) and molecularly cloned (Lin et al. (1985) Proc. Natl. Acad. Sci. USA 82: 7580), and recombinant hEPO has been used successfully in the treatment of anemia due to end stage renal disease. Clinical use of EPO has also been reported in the treatment of anemia associated with AIDS, rheumatoid arthritis, hematological malignancies, and prematurity, and to increase the yield of autologous blood collected preoperatively.

Recombinant hEPO has a molecular mass of 30.4 kD, of which 40% is carbohydrate. Studies of the nature and function of the glycosylation of EPO have determined that the majority of the oligosaccharide chains of hEPO are fucose-containing, sialylated tetraantennary oligosaccharides. The glycosylation structure of human urinary EPO and recombinant EPO produced in Chinese hamster ovary (CHO) cells, baby hamster kidney (BHK) cells and human B-lymphoblastic cells is similar but not identical.

Glycosylation appears to play a role in solubility, biosynthesis and secretion of EPO, and in vivo metabolism of EPO. The in vivo metabolic role of glycosylation, and in particular the presence of sialic acid, has been investigated. It has been demonstrated that urinary and recombinant desialylated EPO lose in vivo activity due to rapid hepatic clearance. From these and similar studies it has been determined that sialic acid caps the penultimate galactose residue and thus protects EPO from clearance by hepatic asialoglycoprotein (galactosyl) receptors. Accordingly, loss of terminal sialic acid residues from the oligosaccharides of EPO exposes the galactose residues, resulting in rapid clearance of desialylated (also referred to as asialylated) EPO from the plasma via binding to the hepatic receptor.

Although desialylated EPO is essentially biologically inactive in vivo due to its rapid clearance, its bioactivity is maintained in established in vitro assays. Standard assays for determining bioactivity of EPO in vitro include measurement of incorporation of tritiated thymidine by splenic erythroblasts from phenylhydrazine-treated anemic mice (Krystal (1983) Exp. Hematol. 20 : 649) or cells of a human pluripotent leukemia cell line (Lewis et al. (1989) Exp. Hematol. 17: 102), measurement of incorporation of ⁵⁹Fe into cultured bone marrow cells (Goldwasser et al. (1975) Endocrinology 97: 315), and measurement of the growth of EPO-dependent cell lines (Kitamura et al. (1989) Blood 73: 375).

Because the in vitro assays cannot discriminate between sialylated and desialylated EPO, such assays are not useful in quantitating the population of EPO which would be expected to be active in vivo. For example, if an EPO-containing sample also contains a significant amount of desialylated EPO, the in vitro assays would provide an overestimate of the in vivo activity. Accordingly, in vivo assays are used to measure the in vivo activity of EPO. Specifically, EPO activity is measured by incorporation of ⁵⁹Fe into erythroblasts of polycythemic mice (Cotes et al. (1961) Nature 191: 1065) or starved rats (Goldwasser et al. (1975) Methods Enzymol. 37: 109).

In a currently used assay which is a modification of the procedure of Cotes et al., female mice are exposed to hypobaric pressure for fourteen days. Endogenous red cell formation is suppressed by the polycythemia produced through exposure to reduced pressure. As the polycythemic state persists after hypoxia, any new red blood cell formation is attributable to the administration of exogenous EPO. The test samples and EPO standards are then injected subcutaneously into the conditioned mice. Forty-eight hours after EPO injection, ⁵⁹Fe is administered. Blood samples are drawn after another forty-eight hours, and radioactivity is quantitated. The quantity of radioactivity is directly proportional to the injected dose of standard EPO; the in vivo activity of unknown samples is calculated from a standard curve.

The in vivo bioassay is widely recognized as the only true measure of in vivo biological activity, since it measures both the circulating life and proliferative activity of EPO. However, the in vivo assay suffers from significant disadvantages in that it is labor-intensive, expensive, time-consuming, and subject to animal-to-animal variation.

The present invention provides an in vitro method of quantitating the in vivo activity of EPO which overcomes the disadvantages of the prior art methods.

### SUMMARY OF THE INVENTION

The present invention is directed to an in vitro method for determining the in vivo EPO activity of a sample containing EPO. More particularly, the present method comprises treating a sample containing EPO under conditions which remove desialylated EPO, and measuring in vitro the EPO activity of the resulting treated sample. In a preferred embodiment, desialylated EPO is removed from the sample by incubating the sample with cells of the human hepatoma cell line HepG2, and in vitro EPO activity is determined by incubating the treated sample with cells of an EPO-responsive cell line and measuring the proliferation or viability of the EPO-responsive cells.

Another aspect of the invention provides a kit which comprises a first container containing cells which express the asialoglycoprotein receptor, and a second container which contains EPO-responsive cells. In another embodiment, the kit further comprises a third container containing a viability indicating dye, such as 3-[4,5-dimethylthiazol-2-yl]-2,5-diphenyltetrazolium bromide (MTT).

### DETAILED DESCRIPTION OF THE INVENTION

The present invention is directed to an in vitro method for determining the in vivo EPO activity of a sample containing EPO. More particularly, the present method comprises treating a sample containing EPO under conditions which remove desialylated EPO, and measuring in vitro the EPO activity of the resulting treated sample. The present invention is particularly useful in determining the in vivo activity of EPO formulations intended for clinical use.

In vitro EPO activity is defined as the ability to stimulate the proliferation or viability, in vitro, of cells which express a functional EPO-receptor, for example, erythroid precursor cells. As discussed hereinbelow, both sialylated and desialylated EPO are active in vitro. In vivo EPO activity is defined as the ability to stimulate the proliferation of erythroid precursor cells in vivo. Desialylated EPO is considered herein to be essentially inactive in vivo due to rapid hepatic clearance.

In accordance with the present invention, an EPO-containing sample can be particulate or liquid, and preferably is serum or plasma, cell culture media, purified or partially purified recombinant EPO, or an EPO formulation intended for clinical use, stability or formulation studies.

According to the present invention, the EPO-containing sample is treated under conditions which remove desialylated EPO. Desialylation of EPO results in the exposure of the penultimate galactose residues of the oligosaccharides of EPO. Accordingly, desialylated EPO can be removed from an EPO-containing sample by subjecting the sample to affinity chromatography with a lectin which has affinity for galactose, for example Abrin A or by affinity chromatography with an immobilized antibody specific for galactose. Methods for performing lectin affinity chromatography are known to the ordinarily skilled artisan.

In another embodiment, desialylated EPO is removed from an EPO-containing sample by incubation with cells which express the asialoglycoprotein receptor. The cells may naturally express the asialoglycoprotein receptor, or may be engineered to express the receptor recombinantly. For example, fibroblasts transfected with cDNA encoding the HL-1 and HL-2 subunits of the asialoglycoprotein receptor express a functional receptor. Transfected fibroblasts expressing the recombinant asialoglycoprotein receptor can be obtained by following the procedure of Shia et al. (1989) Proc. Natl. Acad. Sci. USA 86: 1158.

In a preferred embodiment, desialylated EPO is removed from an EPO-containing sample by incubation with cells of the human hepatoma cell line HepG2 (ATCC No. HB 8065), which are known to naturally express a functional asialoglycoprotein receptor at high density (see, for example, Lodish, (1991) Trends in Biochem. Sciences 16: 374). The HepG2 cell line is described by Knowles et al. (1980) Science 209: 497 and in U.S. Patent 4,393,133 to Knowles et al.

Appropriate conditions for incubating the EPO-containing sample with cells which express the asialoglycoprotein receptor can be determined by the skilled artisan and may vary depending upon the cell type and source of the sample. In one embodiment, cells are grown to sub-confluence, washed, and cell suspensions are added to wells of a tissue culture plate. Each well containing the adherent cells is washed several times with buffer, buffer is removed, and EPO standards or test samples are added to each well. The cells and EPO-containing samples are incubated overnight (about 15-20 hours) at 37°C in a humidified atmosphere of 5%CO₂ and 95% air. The supernatants from the incubation mixtures, which are termed the "treated samples" in accordance with the present invention and from which desialylated EPO has been adsorbed, are subsequently transferred to vessels for analysis of EPO proliferative activity.

In order to confirm that desialylated EPO has been removed either by affinity chromatography or incubation with cells expressing the asialoglycoprotein receptor, EPO can be subjected to desialylating conditions. Chemical and enzymatic methods for desialylation are known to the ordinarily skilled artisan. Briefly, EPO can be chemically desialylated by heating at 80°C for 60 minutes in 0.1 mol/L HCl. Desialylation can also be accomplished by incubating EPO with immobilized neuraminidase. Specific conditions for desialylation of EPO are described by Spivak et al. (1989) Blood 73: 90. An analysis of EPO before and after desialylation, for example by isoelectic focusing, can be used to determine whether sialic acid has been removed.

Following treatment of the EPO-containing sample to adsorb desialylated EPO as described above, the EPO proliferative activity of the treated sample is assessed. As described above, the term "treated sample" refers to a sample which has been treated to remove desialylated EPO, for example by lectin affinity chromatography or by incubation with cells which express a functional asialoglycoprotein receptor. The treated sample can be, for example, a cell culture supernatant.

The proliferative activity can be determined, for example, by measuring changes in cell number or DNA synthesis of EPO-responsive cells in response to incubation with the treated sample. In accordance with the present invention, EPO-responsive cells are defined as cells which proliferate or retain viability in response to EPO. EPO-responsive cells which can be used to measure the in vitro proliferative activity of EPO include erythroid progenitor cells freshly explanted from hematopoietic organs, established cell lines which naturally express EPO receptors, and established cell lines which have been engineered to express the EPO receptor gene. Cell lines which naturally express EPO receptors are generally derived from malignant cells of tumor-bearing animals. For example, murine erythroid cell lines have been established from erythroleukemic mice infected with the Friend leukemia virus complex or the Rauscher leukemia virus complex. Human cells lines have been established from leukemic patients. Other cell lines have been engineered to produce large numbers of EPO receptors and to require EPO for survival by introduction and expression of the EPO receptor gene. EPO-responsive cell systems are known to the ordinarily skilled artisan and have been reviewed by Koury et al. (1992) Eur. J. Biochem. 210: 649.

In a preferred embodiment, the EPO-responsive cells are cells of the multipotential hematopoietic progenitor cell line B6SUtA described by Greenberger et al. (1983) Proc. Natl. Acad. Sci. USA 80: 2931. The B6SUtA cell line was derived from long-term bone marrow cultures of B6.S mice.

Proliferation of EPO responsive cells can be measured by quantitating increased DNA synthesis in response to EPO. Increased DNA synthesis is typically determined in vitro by measuring tritiated thymidine incorporation. Appropriate assays are known in the art and described, for example, by Krystal et al. (1983) Exp. Hematol. 11: 649 and Lewis et al. (1989) Exp. Hematol. 17; 102. Briefly, preparations of EPO-responsive cells are incubated with EPO-containing test samples in microtiter plate wells, typically for 22 hours at 37°C in a humidified atmosphere of 5%CO₂ and 95% air. Tritiated thymidine is added to a final concentration of about 0.1 *µ*Ci/well. Following an additional two hour incubation, well contents are collected on glass fiber filters and the radioactivity recovered is measured by scintillation spectrometry. For standardization, the amount of radioactivity incorporated into DNA is plotted versus the EPO activity in mU/ml.

In a preferred embodiment which avoids the use of radioactivity, proliferation of EPO-responsive cells is measured spectrophotometrically through the use of the dye MTT (3-[4,5-dimethylthiazol-2-yl]-2,5-diphenyl tetrazolium bromide) (Mossman, 1983, Immunological Methods 65, 55). MTT is a yellowish color in solution, and is converted to dark blue/purple water-insoluble MTT formazin by mitochondrial dehydrogenases of living cells. The blue crystals are solubilized with acidic isopropanol and intensity is measured colorimetrically at 570 and 690 nm. Accordingly, the amount of MTT converted to the colored formazin is a direct measure of cell number which is an indicator of cell proliferation. A comparison of the optical densities of unknowns to standards is used to calculate biological activity. In a preferred embodiment, the treated sample is incubated with EPO-responsive cells in the wells of a microtiter plate for about 46-50 hours at 37°C in a humidified atmosphere of 5%CO₂ and 95% air. MTT is then added to each well and incubated for two hours at 37°C. Following solubilization of the blue crystals, optical densities are measured and EPO activity is determined by comparing the cell numbers produced by an unknown sample to an EPO standard curve. MTT is commercially available, as are kits which contain MTT dye and solubilization solution (MTT Kit, Promega Corporation). In addition to MTT, other indicator dyes such as XTT (3,3'-(1-((phenylamino)carbonyl)-3,4-tetrazolium)-bis(4-methoxy-6-nitro) benzenesulfonic acid, sodium salt) and MTS (3-(4,5-dimethylthiazol-2-yl)-5-(3-carboxymethoxyphenyl)-2-(4-sulfophenyl)-2H-tetrazolium, inner salt) may be used for the same purpose.

In a preferred embodiment of the method of the present invention, desialylated EPO is removed from the sample by incubating the sample with cells of the human hepatoma cell line HepG2, and in vitro EPO activity is determined by incubating the treated sample, i.e. the supernatant from the HepG2 cells, with cells of an EPO-dependent cell line, and measuring the proliferation or viability of the EPO-dependent cells. In a most preferred embodiment which is exemplified hereinbelow at Example 1, the EPO-dependent cell line is B6SUtA and cell proliferation or viability is measured spectrophotometrically through the use of MTT.

It has been found in accordance with the present invention that the in vitro affinity of the asialoglycoprotein receptor of HepG2 cells for desialylated EPO effectively mimics the in vivo function of rapid clearance of desialylated EPO. Further, it has been found that cell culture media which contains EPO can induce proliferation of EPO-responsive cells.

Another aspect of the present invention provides a kit for determining the in vivo EPO activity of a sample containing EPO. In one embodiment, the kit is compartmentalized to receive a first container containing cells which express the asialoglycoprotein receptor, and a second container which contains EPO-dependent cells. In a preferred embodiment, the cells which express the asialoglycoprotein receptor are HepG2 cells and the EPO-dependent cells are B6SUtA cells. In another embodiment, the kit further comprises a third container which contains MTT.

The following example further illustrates the present invention. The invention is not to be considered limited by these examples, but only by the appended claims.

### EXAMPLE 1

HepG2 cells are grown in 100 mm tissue culture plates until confluence is reached. At this point the cells are washed with phosphate buffered saline, trypsinized to detach them from the plate and a 0.25 x 10⁶ cells/ml suspension in cell culture medium is made. One ml of this suspension is added to each well of a 24-well tissue culture plate. Three days later the HepG2 cells are ready to be used in an EPO assay.

Each well containing HepG2 cells is washed five times with phosphate buffered saline (PBS). After the PBS is entirely removed, 100 *µ*l of medium (Dulbecco's MEM containing 10% fetal calf serum and glutamine) is added to each well. An EPO standard curve is initiated by taking an EPO reference standard at 10 U/ml and diluting this two-fold by mixing 100 µl of each solution added to a separate well. The cells plus EPO solutions are incubated overnight at 37°C in a 5% CO₂ high humidity incubator. After incubation, the standard curve is prepared by diluting the HepG2-treated 5 U/ml standard five-fold in medium to give a 1 U/ml solution. Each treated sample is diluted 10-fold in medium to give estimated 0.5 U/ml solutions. The standards and samples are then ready for the proliferation assay. At about the same time that the standard and samples are added to the HepG2 cells, B6SUtA cells are prepared for the proliferation assay to be started the next day. These cells have been growing in medium (as described above) and supplemented with 20 ng/ml IL-3; passaged twice per week at a 1:20 dilution. The cells are prepared by washing once with medium and reconstituted to the original volume in medium without IL-3. The cells are incubated overnight in 100 mm plates (37°C, 5% CO₂). After incubation, the cells are washed again in medium and resuspended at a final concentration of 1 x 10⁶ cells/ml. Using a 96-well tissue culture plate, 50 µl of B6SUtA cells are mixed with 50 µl of each EPO standard and sample. The plate is incubated 48 hours at 37°C in a 5% CO₂ incubator.

Cell proliferation or viability is measured with the MTT Kit from Promega Corp. The amount of MTT converted to a colored formazin is directly related to the number of cells. The procedure, in brief is as follows. Twenty µl of MTT dye (Promega Corp.) is added to each well and incubated for 2 hours at 37°C. Then 100 µl of the Solubilization Solution (Promega Corp.) is added to each well and incubated at room temperature until all the dark blue/purple precipitate dissolves, usually 1-4 hours. The plate is then read in a plate reader at O.D. 570 nm and 690 nm. EPO activity is determined by comparing the number of cells produced by an unknown sample to the EPO standard curve. Potency is calculated by dividing calculated activity by 0.5 U/ml and multiplying by 100%.

Table 1 presents representative optical densities and calculated values of EPO standards ranging in concentration from 0-1000 mU/ml determined under the above conditions. As can be seen from the calculated values, the method of the present invention provides an accurate measurement of the EPO standards.

When the same standards are assayed for proliferative activity in the absence of pre-incubation with HepG2 cells, similar results are obtained, as presented in Table 2. These results indicate that the HepG2 cell treatment does not significantly affect the EPO standard curve.

Samples with known and varying amounts of desialylated EPO were assayed by the present method and also in the absence of pre-incubation with HepG2 cells. As can be seen from Table 3, samples containing significant amounts of desialylated EPO induce a greater proliferative response before incubation with HepG2 cells (R2) than after incubation with HepG2 cells and adsorption of desialylated EPO (R1). Table 3 also provides the expected quantities of sialylated (intact) EPO and the values calculated from the optical densities obtained in accordance with the present method. As can be seen from Table 3, the present method is highly accurate in quantitating the amount of sialylated (i.e. in vivo active) EPO in a sample.

The present invention has been described herein with reference to certain preferred embodiments and an example. Since obvious variations will appear to those skilled in the art, the invention is not to be considered limited thereto, but only by the claims which follow.

**TABLE 1**

| EPO Activity of Standards Following Pre-Incubation with HEPG2 Cells | | | | | | | |
|---|---|---|---|---|---|---|---|
| Standard | Std. Value | | OD | Mean | Std. Dev. | CV | Calc. Value |
| STD00 | 1000 | mU/ml. | 1.304 | 1.336 | 0.029 | 2.151 | 942.9 |
| | | | 1.359 | | | | 1080 |
| | | | 1.346 | | | | 1044 |
| STD01 | 900.0 | mU/ml. | 1.244 | 1.271 | 0.057 | 4.494 | 824.6 |
| | | | 1.233 | | | | 805.6 |
| | | | 1.337 | | | | 1021 |
| STD02 | 800.0 | mU/ml. | 1.223 | 1.240 | 0.037 | 2.951 | 788.9 |
| | | | 1.215 | | | | 775.9 |
| | | | 1.282 | | | | 896.4 |
| STD03 | 700.0 | mU/ml. | 1.133 | 1.161 | 0.040 | 3.410 | 659.9 |
| | | | 1.143 | | | | 672.6 |
| | | | 1.206 | | | | 761.7 |
| STD04 | 600.0 | mU/ml. | 1.114 | 1.109 | 0.019 | 1.713 | 636.6 |
| | | | 1.088 | | | | 606.6 |
| | | | 1.125 | | | | 649.9 |
| STD05 | 500.0 | mU/ml. | 0.961 | 0.965 | 0.034 | 3.488 | 483.5 |
| | | | 0.933 | | | | 460.5 |
| | | | 1.000 | | | | 517.8 |
| STD06 | 400.0 | mU/ml. | 0.827 | 0.846 | 0.025 | 2.950 | 383.0 |
| | | | 0.874 | | | | 415.6 |
| | | | 0.836 | | | | 389.0 |
| STD07 | 300.0 | mU/ml. | 0.691 | 0.689 | 0.017 | 2.414 | 300.1 |
| | | | 0.704 | | | | 307.4 |
| | | | 0.671 | | | | 289.0 |
| STD08 | 200.0 | mU/ml. | 0.477 | 0.496 | 0.016 | 3.261 | 192.6 |
| | | | 0.505 | | | | 205.6 |
| | | | 0.505 | | | | 205.6 |
| STD09 | 100.0 | mU/ml. | 0.269 | 0.270 | 0.002 | 0.565 | 99.79 |
| | | | 0.270 | | | | 100.2 |
| | | | 0.272 | | | | 101.1 |
| STD10 | 0.000 | mU/ml. | 0.093 | 0.096 | 0.006 | 6.720 | <<<<< |
| | | | 0.091 | | | | <<<<< |
| | | | 0.103 | | | | 10.30 |

**TABLE 2**

| EPO Activity of Standards Without Pre-Incubation with HEPG2 Cells | | | | | | | |
|---|---|---|---|---|---|---|---|
| Standard | Std. Value | | OD | Mean | Std. Dev. | CV | Calc. Value |
| STD00 | 1000 | mU/ml. | 1.439 | 1.517 | 0.069 | 4.566 | 923.1 |
| | | | 1.539 | | | | 1141 |
| | | | 1.572 | | | | 1236 |
| STD01 | 900.0 | mU/ml. | 1.362 | 1.425 | 0.074 | 5.227 | 800.7 |
| | | | 1.405 | | | | 865.4 |
| | | | 1.507 | | | | 1062 |
| STD02 | 800.0 | mU/ml. | 1.264 | 1.364 | 0.097 | 7.122 | 679.2 |
| | | | 1.371 | | | | 813.5 |
| | | | 1.458 | | | | 958.4 |
| STD03 | 700.0 | mU/ml. | 1.251 | 1.278 | 0.054 | 4.240 | 665.2 |
| | | | 1.242 | | | | 655.7 |
| | | | 1.340 | | | | 770.6 |
| STD04 | 600.0 | mU/ml. | 1.177 | 1.185 | 0.007 | 0.599 | 592.8 |
| | | | 1.186 | | | | 601.0 |
| | | | 1.191 | | | | 605.6 |
| STD05 | 500.0 | mU/ml. | 1.071 | 1.083 | 0.011 | 1.028 | 505.9 |
| | | | 1.085 | | | | 516.4 |
| | | | 1.093 | | | | 522.6 |
| STD06 | 400.0 | mU/ml. | 0.917 | 0.902 | 0.019 | 2.061 | 403.7 |
| | | | .907 | | | | 397.8 |
| | | | 0.881 | | | | 382.8 |
| STD07 | 300.0 | mU/ml. | 0.724 | 0.744 | 0.035 | 4.732 | 300.8 |
| | | | 0.785 | | | | 331.1 |
| | | | 0.724 | | | | 300.8 |
| STD08 | 200.0 | mU/ml. | 0.435 | 0.455 | 0.017 | 3.813 | 173.6 |
| | | | 0.464 | | | | 185.7 |
| | | | 0.466 | | | | 186.5 |
| STD09 | 100.0 | mU/ml. | 0.271 | 0.281 | 0.009 | 3.225 | 104.6 |
| | | | 0.288 | | | | 112.0 |
| | | | 0.285 | | | | 110.7 |
| STD10 | 0.000 | mU/ml. | 0.096 | 0.097 | 0.003 | 2.728 | <<<<< |
| | | | 0.095 | | | | <<<<< |
| | | | 0.100 | | | | 4.484 |

**TABLE 3**

| Quantitation of Intact (Sialylated) EPO | | | | | | | |
|---|---|---|---|---|---|---|---|
| % Asialo EPO | R1 w/ HepG-2 mU/ml | R2 w/o/ HepG-2 mU/ml | R2 /R1 Ratio | k=0.15 Calculated Intact EPO mU/ml | k=0.15 Calculated % Intact EPO | Expected Intact EPO mU/ml | % Accuracy |
| 0.00 | 289.10 | 303.60 | 1.05 | 286.54 | 1.00 | 300.00 | 0.96 |
| 10.00 | 268.60 | 352.80 | 1.31 | 253.74 | 0.89 | 270.00 | 0.94 |
| 20.00 | 261.70 | 424.20 | 1.62 | 233.02 | 0.81 | 240.00 | 0.97 |
| 30.00 | 254.70 | 494.20 | 1.94 | 212.44 | 0.74 | 210.00 | 1.01 |
| 40.00 | 217.60 | 555.40 | 2.55 | 157.99 | 0.55 | 180.00 | 0.88 |
| 50.00 | 214.60 | 628.30 | 2.93 | 141.59 | 0.49 | 150.00 | 0.94 |
| 60.00 | 213.40 | 693.60 | 3.25 | 128.66 | 0.45 | 120.00 | 1.07 |
| 70.00 | 192.30 | 755.50 | 3.93 | 92.91 | 0.32 | 90.00 | 1.03 |
| 80.00 | 196.10 | 959.90 | 4.89 | 61.31 | 0.21 | 60.00 | 1.02 |
| 90.00 | 178.60 | 999.20 | 5.59 | 33.79 | 0.12 | 30.00 | 1.13 |
| 100.00 | 150.80 | 1029.00 | 6.82 | -4.18 | -0.01 | 0.00 | #DIV/ 0! |

## Claims

1. A method for the in vitro determination of in vivo erythropoietin (EPO) activity in an EPO-containing sample, which comprises treating the sample under in vitro conditions which remove desialylated EPO, and measuring the in vitro EPO activity of the treated sample.

2. The method of Claim 1, wherein said in vitro conditions which remove desialylated EPO comprise lectin affinity chromatography.

3. The method of Claim 1, wherein said in vitro conditions which remove desialylated EPO comprise incubation with cells which express an asialoglycoprotein receptor.

4. The method of Claim 3, wherein said cells express said asialoglycoprotein receptor naturally or recombinantly.

5. The method of Claim 3, wherein said cells are HepG2 cells.

6. The method of Claim 1, wherein said in vitro EPO activity is measured by incubating said treated sample with EPO-responsive cells and measuring proliferation or viability of said EPO-responsive cells.

7. The method of Claim 6, wherein said EPO-responsive cells are B6SUtA cells.

8. The method of Claim 6, wherein said proliferation of said EPO-responsive cells is measured by determining an increase in DNA synthesis.

9. The method of Claim 8, wherein said increase in DNA synthesis is measured by determining tritiated thymidine incorporation.

10. The method of Claim 6, wherein said proliferation of said EPO-responsive cells is measured spectrophotometrically.

11. The method of Claim 6, wherein said proliferation of said EPO-responsive cells is measured by incubating said cells with 3-[4,5-dimethylthiazol-2-yl]-2,5-diphenyltetrazolium bromide (MTT) and measuring the conversion of MTT to MTT formazin.

12. A method for the in vitro determination of in vivo erythropoietin EPO activity in an EPO-containing sample, which comprises: (a) incubating said sample with HepG2 cells; (b) removing the supernatant from said HepG2 cells; (c) incubating said supernatant with B6SUtA cells; (d) measuring proliferation of said B6SUtA cells; and (e) calculating EPO activity.

13. The method of Claim 12, wherein said sample containing EPO is incubated with said HepG2 cells for about 15 to 20 hours at 37°C in a humidified atmosphere of 5% CO₂ and 95% air.

14. The method of Claim 12, wherein said supernatant in incubated with said B6SUtA cells for about 46 to 50 hours at 37°C.

15. The method of Claim 12, wherein said proliferation of said B6SUtA cells is measured by incubating said cells with MTT and measuring the conversion of MTT to MTT formazin.

16. The method of Claim 12, wherein said EPO activity is calculated by comparing the proliferation produced by the EPO-containing sample to the proliferation produced by an EPO standard.

17. A compartmentalized kit, comprising a first container containing cells which express an asialoglycoprotein receptor and a second container containing EPO-responsive cells.

18. The compartmentalized kit of Claim 17, wherein said cells which express an asialoglycoprotein receptor are HepG2 cells.

19. The compartmentalized kit of Claim 17, wherein said EPO-responsive cells are B6SUtA cells.

## Patentansprüche

1. Verfahren zur *in vitro*-Bestimmung der Aktivität von Erythropoietin (EPO) *in vivo* in einer EPO-enthaltenden Probe, die Behandeln der Probe unter *in vitro*-Bedingungen, die desialinyliertes EPO entfernen, und Messen der *in vitro* EPO-Aktivität in der behandelten Probe umfaßt

2. Verfahren nach Anspruch 1, wobei die *in vitro*-Bedingungen, die desialinyliertes EPO entfemen, Lectin-Affinitätschromatographie umfassen.

3. Verfahren nach Anspruch 1, wobei die *in vitro*-Bedingungen, die desialinyliertes EPO *in vitro* entfemen, eine Inkubation mit Zellen umfassen, die einen Asialoglycoprotein-Rezeptor exprimieren.

4. Verfahren nach Anspruch 3, wobei die Zellen den Asialoglycoprotein-Rezeptor natürlich oder rekombinant exprimieren.

5. Verfahren nach Anspruch 3, wobei die Zellen HepG2-Zellen sind.

6. Verfahren nach Anspruch 1, wobei die *in vitro* EPO-Aktivität durch Inkubieren der behandelten Probe mit auf EPO-ansprechenden Zellen und Messen der Proliferation oder Lebensfähigkeit der genannten auf EPO-ansprechenden Zellen gemessen wird.

7. Verfahren nach Anspruch 6, wobei die auf EPO-ansprechenden Zellen B6SUtA-Zellen sind.

8. Verfahren nach Anspruch 6, wobei die Proliferation der auf EPO-ansprechenden Zellen durch Bestimmen einer Zunahme der DNA-Synthese gemessen wird.

9. Verfahren nach Anspruch 8, wobei die Zunahme der DNA-Synthese durch Bestimmen des Einbaus von Tritium-markiertem Thymidin gemessen wird.

10. Verfahren nach Anspruch 6, wobei die Proliferation der auf EPO-ansprechenden Zellen spektrophotometrisch gemessen wird.

11. Verfahren nach Anspruch 6, wobei die Proliferation der auf EPO-ansprechenden Zellen durch Inkubieren der Zellen mit 3-[4,5-Dimethylthiazol-2-yl]-2,5-Diphenyltetrazolium Bromid (MMT) und Messen des Umsatzes von MTT zu MTT-Formazin gemessen wird.

12. Verfahren zur *in vitro*-Bestimmung der *in vivo* Erythropoietin EPO-Aktivität in einer EPO-enthaltenden Probe, die umfaßt: (a) Inkubieren der Probe mit HepG2-Zellen; (b) Entfernen des Überstands von den HepG2-Zellen; (c) Inkubieren des Überstands mit B6SUtA-Zellen; (d) Messen der Proliferation der B6SUtA-Zellen; und (e) Berechnen der EPO-Aktivität.

13. Verfahren nach Anspruch 12, wobei die EPO-enthaltende Probe mit den HepG2-Zellen ungefähr 15 bis 20 Stunden bei 37°C in einer befeuchteten Atmosphäre von 5% CO₂ und 95% Luft inkubiert wird.

14. Verfahren nach Anspruch 12, wobei der genannte Überstand mit den B6SUtA-Zellen ungefähr 46 bis 50 Stunden bei 37°C inkubiert wird.

15. Verfahren nach Anspruch 12, wobei die Proliferation der B6SUtA-Zellen durch Inkubieren der Zellen mit MTT und Messen des Umsatzes von MTT zu MTT-Formazin gemessen wird.

16. Verfahren nach Anspruch 12, wobei die EPO-Aktivität durch Vergleichen der durch die EPO-enthaltende Probe produzierten Proliferation, mit der Proliferation, die durch einen EPO-Standard produziert wird, berechnet wird.

17. Kompartimentierter Testkit, umfassend einen ersten Behälter, der Zellen, die einen Asialoglycoprotein-Rezeptor exprimieren, enthält, und einen zweiten Behälter, der auf EPO-ansprechende Zellen enthält.

18. Kompartimentierter Testkit nach Anspruch 17, wobei die Zellen, die einen Asialoglycoprotein-Rezeptor exprimieren, HepG2-Zellen sind.

19. Kompartimentierter Testkit nach Anspruch 17, wobei die auf EPO-ansprechenden Zellen B6SUtA-Zellen sind.

## Revendications

1. Méthode pour la détermination in vitro de l'activité d'érythropoïétine (EPO) in vivo dans un échantillon contenant EPO, qui comprend le traitement de l'échantillon en conditions in vitro qui éliminent EPO désialylée, et la mesure de l'activité de EPO in vitro de l'échantillon traité.

2. Méthode de la revendication 1, où lesdites conditions in vitro qui éliminent EPO désialylée comprennent une chromatographie par affinité de lectine.

3. Méthode de la revendication 1, où lesdites conditions in vitro qui éliminent EPO désialylée comprennent une incubation avec des cellules qui expriment un récepteur d'asialoglycoprotéine.

4. Méthode de la revendication 3, où lesdites cellules expriment ledit récepteur d'asialoglycoprotéine naturellement ou de manière recombinante.

5. Méthode de la revendication 3, où lesdites cellules sont des cellules HepG2.

6. Méthode de la revendication 1, où ladite activité de EPO in vitro est mesurée par incubation dudit échantillon traité avec des cellules sensibles à EPO et mesure de la prolifération ou de la viabilité desdites cellules sensibles à EPO.

7. Méthode de la revendication 6, où lesdites cellules sensibles à EPO sont des cellules B6SUtA.

8. Méthode de la revendication 6, où ladite prolifération desdites cellules sensibles à EPO est mesurée en déterminant une augmentation de la synthèse d'ADN.

9. Méthode de la revendication 8, où ladite augmentation de la synthèse d'ADN est mesurée en déterminant l'incorporation de thymidine tritiée.

10. Méthode de la revendication 6, où ladite prolifération desdites cellules sensibles à EPO est mesurée spectrophotométriquement.

11. Méthode de la revendication 6, où ladite prolifération desdites cellules sensibles à EPO est mesurée par incubation desdites cellules avec du bromure de 3-[4,5-diméthylthiazol-2-yl]-2,5-diphényltétrazolium (MTT) et mesure de la conversion de MTT en MTT formazine.

12. Méthode pour la détermination in vitro de l'activité de l'érythropoïétine EPO in vivo dans un échantillon contenant EPO qui comprend: (a) l'incubation dudit échantillon avec des cellules HepG2; (b) l'élimination du fluide surnageant desdites cellules HepG2; (c) l'incubation dudit fluide surnageant avec des cellules B6SUtA; (d) la mesure de la prolifération desdites cellules B6SUtA; et (e) le calcul de l'activité de EPO.

13. Méthode de la revendication 12, où ledit échantillon contenant EPO est soumis à incubation avec lesdites cellules HepG2 pendant environ 15 à 20 heures à 37°C dans une atmosphère humidifiée de 5% CO₂ et 95% air.

14. Méthode de la revendication 12, où ledit produit surnageant est soumis à incubation avec lesdites cellules B6SUtA pendant environ 46 à 50 heures à 37°C.

15. Méthode de la revendication 12, où ladite prolifération desdites cellules B6SUtA est mesurée par incubation desdites cellules avec MTT et mesure de la conversion de MTT en MTT formazine.

16. Méthode de la revendication 12, où ladite activité de EPO est calculée en comparant la prolifération produite par l'échantillon contenant EPO avec la prolifération produite par un standard de EPO.

17. Kit compartimentalisé, comprenant un premier conteneur contenant des cellules qui expriment un récepteur d'asialoglycoprotéine et un second conteneur contenant des cellules sensibles à EPO.

18. Kit compartimentalisé de la revendication 17, où lesdites cellules qui expriment un récepteur d'asialoglycoprotéine sont des cellules HepG2.

19. Kit compartimentalisé de la revendication 17, où lesdites cellules sensibles à EPO sont des cellules B6SUtA.
